# EUROPEAN PATENT SPECIFICATION

(11) **EP 4 298 201 B1**
(45) Date of publication and mention of the grant of the patent: **30.04.2025**
(21) Application number: 22712043.3
(22) Date of filing: 25.02.2022
(51) Int. Cl.: C12M 3/00, B01L 3/00, B01L 9/00, C12M 3/06

(54) **MULTIORGAN-ON-A-CHIP DEVICE WITH INTEGRATED MICROBIOSENSORS, METHODS AND USES THEREOF**
MULTIORGAN-ON-A-CHIP-VORRICHTUNG MIT INTEGRIERTEN MIKROBIOSENSOREN, VERFAHREN UND VERWENDUNGEN DAVON
DISPOSITIF MULTI-ORGANE SUR PUCE AVEC MICROBIOCAPTEURS INTÉGRÉS, PROCÉDÉS ET UTILISATIONS DE CE CELUI-CI

(30) Priority: 26.02.2021 PT 2021117089
(43) Date of publication of application: 03.01.2024
(73) Proprietor: Universidade do Minho, 4704-553 Braga (PT); International Iberian Nanotechnology Laboratory (INL), 4715-330 Braga (PT)
(72) Inventor: HENRIQUES MINAS, Graça Maria, 4710-588 Braga (PT); OLIVEIRA RODRIGUES, Raquel, 4820-179 Fafe (PT); TEIXEIRA DE SOUSA, Paulo Jorge, 4705-769 Braga (PT); MADEIRA MACEDO DE LIMA, Rui Alberto, 4715-586 Braga (PT); SARAIVA DE SOUSA, Patrícia Catarina, 4470-593 Maia (PT); CALAZA CABANAS, Carlos Alberto, 4715-363 Braga (PT)
(74) Representative: Patentree
(86) International application number: PCT/IB2022/051689
(87) International publication number: WO 2022/180595

(56) References cited:
- WO-A1-2013/086486
- WO-A1-2013/086502
- WO-A1-2018/140497
- WO-A1-2018/213357
- US-A1- 2018 272 346
- US-A1- 2020 369 996

## Description

### TECHNICAL FIELD

The present disclosure relates to a device to produce an organoid cell culture or cultures, preferably a multi organ-on-a-chip, and the use thereof.

The present disclosure relates to an advanced microfluidic system for the validation and study of nanomaterials, drugs, or mixtures thereof, intended to be used in biomedical and/or pharmaceutic applications. The microfluidic system allows the determination of the toxicological and/or therapeutic effect in three-dimensional (3D) models of human organs, with the integration of micro(bio)sensors directly on the cell models, as well as in the microfluidic system to monitor the feed fluid enabling the automatic replacement when needed.

### BACKGROUND

Raising from the advance of nanotechnology, the nanomedicine that applies nanomaterials as multifunctional stimuli-responsive drug nanocarriers triggered in response at different physicochemical parameters, such as pH and temperature, is being developed with great optimism to treat and diagnose emergent diseases. In particular, cancer tissues have a more acidic pH than the healthy ones. Therefore, the same nanomaterial can be designed as pH-dependent drug delivery nanocarrier and simultaneously incorporate other therapeutic properties, such as hyperthermia, gene therapy, among others. However, until now, the standard preclinical methodologies to screen those nanomaterials fail into mimic the complex physiological microenvironment found in the human body, and thus the validation of these nanosystems could not be clearly proven, causing the failing in the applicability of nanomedicine.

Organ-on-chip (OoC) platforms is a game-changing technology that is able to mimic complex human organ functions and realistic (patho)physiology at the microscale level. This technology promises to substitute animal models for the analysis of drug efficacy, toxicity, pharmacokinetics and pharmacodynamics. More recently, this technology is also being suggested as a platform for the preclinical evaluation of nanomaterials developed for medicine, with application in the diagnosis and/or therapy (the so-called theranostic).

Although their potential, the end-use of OoC platform has been limited by the successful integration of multi(bio)sensors that fully capture the complexities of *in vivo* metabolism in a robust manner and, the combination of multi-OoC (MoC) for the systemic interaction and homeostasis (auto-regulation) of several organ models in a single system. Ideally, MoC should include integrated multiplexed (bio)sensors to monitor various physical/biochemical parameters occurring in the organ models, such as O₂, CO₂, temperature, pH, released biomarkers and others. This integrated multiplexed (bio)sensor system allows the real-time monitoring of the cellular viability of the organ models, cellular growth, maintenance, as well as the evaluation of the toxicological effect of drugs and nanomaterials developed for biomedical applications.

Since the pioneering work, in 2010, of the research group led by Donald Ingber, Wyss Institute (Boston, MA, USA), where the concept of OoC was introduced with the development of a lung-on-a-chip (Huh et al., "Reconstituting lung functions at the organ level on a chip", Science, 2010, 328: 1662-1668 (10.1126/science.1188302)), several other organ models have been described in the literature, showing the potential of these advanced microfluidic devices for diagnosis and treatment of human diseases (Rodrigues et al., "Organ-on-a-chip: A preclinical Microfluidic Platform for the Progress of Nanomedicine", Small, 2020, 2003517 (DOI:10.1002/smll.202003517)). Among the most relevant, the work of Y.S. Zhang et al., ("Organ platform on chips integrated with several sensors for automated and continuous monitoring in situ of organoid behaviors", PNAS, 2017, 114 (12) E2293-E2302 (DOI: 10.1073/ pnas.1612906114)), stands out as a first attempt to integrate an external module of multi-sensors for monitor organ models, describing the implementation of a modular system of physical sensors (pH and temperature) and biochemical (molecular biomarkers) connected externally with OoC for monitoring the antitumor effects of drugs. Another important work was published by S. Shin et al., ("Regenerative, unlabeled electrochemical microfluidic biosensors for continuous monitoring of cellular secretomes", 2017, Adv. Sci. 4: 1600522 (DOI: 10.1002/advs.201600522)), describing an electrochemical biosensor system to monitor biomarkers released by the organ models. However, these documents describe modular platforms connected, but not integrated, with advanced microfluidic devices. Thus, in these systems, the response of organ models to the analysed parameters is post-analysed off-line, which besides time-consuming, is prone to contamination and sample degradation, hampering their feasibility as a standard, autonomous and robust pre-clinical model for laboratory practice.

In the patent document WO2013/086486, it is described an OoC system connected with a commercial micro-clinical analyser sensor chip for the analysis of glucose, oxygen, lactate and pH. However, this is a commercial sensor system connected externally to the biological platform for the analysis of the culture medium circulating in the device. Thus, not providing physiological information on the pH and temperature inside of the biological models, which is essential for the preclinical evaluation of nanomaterials and/or drug studies.

In the document US10078075B2, it is described a MoC composed of a microfluidic system with independent micro-valves, which allows to control and analyse the effect of drugs in various organ models. The device consists of a modular microfluidic system, cell models, pressure sensors and a commercial micro-clinical analyser sensor chip for glucose, oxygen, lactate and pH analysis. However, all sensors are designed for cell culture media analysis and not for the biological models. Once more, this invention does not provide physiological information on the pH and cell temperature of the biological models, which is essential for drug and nanomedicine studies.

In the patent document US2014/0302549A1, it is described an organ-on-chip system, which includes at least one biological model, and can be connected to other biological models, by a capillary network in a microfluidic system. This system supports the integration of one or more sensors, such as temperature, pH, oxygen, biomarkers, among others, connected by microfluidic channels for the analysis of the culture media in circulation. However, this system does not provide direct and individual physiological information on pH and cell temperature of the biological models (it only provides of the cellular medium), which is essential for drug and/or nanomedicine studies.

In the patent documents CA2885375A1, WO2014/048637 and PT2712918E, it is described a MoC system composed of a base layer, an organ layer arranged on the base layer, a connection layer called by the authors as "an antral layer" arranged on the organ layer and an actuator layer. The organ layer allows to host at least one organ model, being configured to comprise a micro-inlet and a micro-outlet for fluid communication of the biological models and a microfluidic system. The base layer also admits to comprise one or more sensors to measure the main homeostasis' parameters of the human organoids, by monitoring the feed medium, which may include, pH sensors, pO₂ sensors, analyte capture sensors, temperature sensors, CO₂ sensors, NO sensors, cytokine sensors, ion sensors, pressure sensors and plasmon resonance sensors. In particular, the invention aims to include 2 pO₂ sensors for measuring oxygen in the fluid of the organ layer; 4 transepithelial/endothelial electrical resistance (TEER) sensors to detect spills in the autonomous circulation system, in which two TEER sensors are located in the autonomous circulation system and two TEER sensors are configured and located in representative organ models; and, electrical sensors, coupled to biological neural nodes. However, this system does not provide direct and individual physiological information on the cellular pH and temperature of the biological models (it only provides of the cellular medium), which is essential for drug and/or nanomedicine studies.

Because cellular pH and local temperature are essential for the homeostasis monitoring of the different organ models in a MoC, the existing solutions, which are based in the integration of microsensors in the microfluidic system for the analysis of the feeding culture media, and not *in situ* integrated within the different multiorgan models, fail into provide a real-time, direct and accurate information on the physiological microenvironment found in each organ model.

These facts are disclosed in order to illustrate the technical problem addressed by the present disclosure.

### GENERAL DESCRIPTION

The invention is defined by a device and the use of the device as defined in claims 1 and 15, respectively. The present disclosure relates to an advanced microfluidic system comprising multiplexed micro(bio)sensors, in situ implemented, with representative organ models, to monitor physicochemical parameters for drug tests and nanomaterial preclinical screening, as well as a pH optical sensor integrated in the microfluidic system to monitor the feeding fluid, enabling the automatic replacement of it when needed, which ensure the long-term cell culture homeostasis. It also comprises a heating system for mimicking hyperthermia treatment.

An aspect of the present disclosure, relates to the insert multiplexed micro(bio)sensors in direct contact with the different organ models, allowing a direct and individual monitoring of important physicochemical parameters, such as pH and temperature, which can be unlike depending on the type of organ model. Thus, this invention gives precise information about the individual organ physiological conditions that are essential for the individual monitoring of their viability and toxicological effect, particularly when subjected to drug tests and nanomaterials developed for medicine. Additionally, it also inserts a heating system for mimicking hyperthermia treatment, adding an important parameter for the preclinical validation of thermo-responsive nanomaterials designed for therapeutic applications.

The present disclosure differs from other approaches by in-situ implementing multi(bio)sensors in contact with representative human organ models, allowing the individual monitoring of each organ model, separately, and at real-time. In addition, and in a complementary way, the device according to the present invention, also comprises at least, an optical pH sensor system for monitoring of the feed fluid circulating in the microfluidic system. By so, the present disclosure allows to monitor the condition necessary for the culture and growth of the organ models in the advanced microfluidic device, but also evaluate individually the viability of each organ model subjected to toxicological tests with drugs and/or nanomaterials.

The microfluidic system of the present disclosure comprises temperature sensors, pH sensors, and a heating system. Thus, the present disclosure refers to a device to produce an organoid cell culture or cultures, preferably a multiorgan-on-a-chip (MoC) device, which mimic the basic functions of one or more organ and/or organism, with the integration of micro(bio)sensors that include, but are not limited to pH and temperature sensors, inserted in the culture perfusion chamber, directly in contact with the human organ models. In addition, a system of heaters is integrated for the local heating of the biological models, representative of human organs, allowing to mimic the temperature of hyperthermia treatment (41 - 45 °C). This heating approach adds an important parameter for the preclinical validation of thermo-responsive nanomaterials designed for therapeutic applications, such as magnetic hyperthermia and/or controlled drug release, without the need to integrate electromagnetic systems that can interfere with the monitoring activity of the remaining (bio)sensors.

The differences of the present disclosure in respect of the prior art include the insertion of micro(bio)sensors for real-time monitoring of individual organ models and a heating system located at the cell culture perfusion chamber for mimicking hyperthermia conditions that enable the preclinical study of thermo-responsive multifunctional nanomaterials developed for biomedical application.

An aspect of the present disclosure, relates to a device for the production of an organoid cell culture or cultures comprising:
a base layer for support;
a transducer layer arranged on the base layer; and
an intermediate layer, arranged on the transducer layer, comprising a plurality of openings to form cavities for organoid cell growth and a plurality of slits to form a microchannel network for fluid flow;
wherein the transducer layer comprises a heating device and at least a plurality of sensors in each cavity for cell parameter control and growth;
wherein each sensor is located to measure an organoid cell growth parameter in the interior of each cavity, preferably near the desired area of organoid cell growth this feature helps to study the evolution of an organoid with a cell mix, for example tumoral and healthy cells.

According to the invention, the device further comprises a heating device located in the interior of each cavity.

According to the invention, the heating device and the plurality of sensors are in direct contact with the organoid cell culture or cultures.

According to the invention, the plurality of sensors is selected from temperature sensor, pH sensor or combinations thereof.

In an embodiment, the sensor may be a temperature sensor, a pH sensor, or combinations thereof. The sensors are distributed uniformly or randomly in the cavity for the detection of the parameter in different points of the organoid.

In an embodiment, the temperature sensors are resistance temperature detectors.

In an embodiment, the numbers of temperature sensors in each cavity are from 1 to 48, preferably 4 and 16.

In an embodiment, the temperature sensor has meander shaped resistor of a metallic film.

In an embodiment, the meander shape comprises 5 to 72 windings, preferably 5 to 24.

In an embodiment, the temperature sensor metallic film is selected from a list consisting of platinum (Pt), nickel (Ni), titanium (Ti), chromium (Cr), gold (Au), aluminium (Al), and their mixtures.

In an embodiment, the configuration of the heating device is selected from meander, spiral, coil, among others. Preferably the configuration is spiral.

In an embodiment, the heating device is made of a metallic film filament, preferably the metallic film is platinum.

In an embodiment, the metallic film filament thickness ranges 50 nm - 200 nm.

The thickness can be obtained for several different measure methods, in the present disclosure was obtained using a mechanical profilometer.

In an embodiment, heater device comprises a plurality of heater lines, preferably wherein the heater line comprises 30 µm - 500 µm of width.

In an embodiment, the heater lines comprise 5000 µm - 50000 µm of length.

In an embodiment, the space between the heater lines ranges from 30 µm - 360 µm.

In an embodiment, the transducer layer comprises a plurality of pH sensors.

In an embodiment, the plurality of pH sensors is grouped, wherein each cavity comprises several groups of a plurality of pH sensors; preferably wherein the plurality of pH sensors is grouped for forming a matrix. In an embodiment, the groups of a plurality of pH sensors are distributed uniformly in the cavity.

In an embodiment, the shape of the pH sensors is a micro-needle shape or a pyramidal shape, preferably a pyramidal shape having a flat bottom surface and a sharp tip.

In an embodiment, the number of pH sensors in each cavity is 4 to 200, preferably 6 to 78.

In an embodiment, the sharp tip of the pyramidal shape comprises 8 high index crystal planes.

In an embodiment, the flat bottom surface of the pyramidal shape comprises {100}-silicon crystal orientation.

In an embodiment, the aspect ratio of the pyramid height to the bottom diameter of the high index crystal planes is 3:2.

In an embodiment, the pH sensors are obtainable from a silicon wafer comprising {100}-crystal orientation on a wafer surface plane, by using an anisotropic silicon wet etch process with potassium hydroxide,

In an embodiment, the method of production of the pH sensors comprises the following steps:
obtaining a silicon wafer surface by depositing a silicon dioxide (SiO₂) layer followed by a silicon nitrite (SiNx) layer, by plasma enhanced chemical vapour deposition;
exposing the silicon wafer surface to hexamethyldisilazane (HDMS);
patterning the silicon wafer surface by means of optical lithography;
transfer the pattern to an oxide/nitrite bi-layer by means of a reactive ion etching process;
obtain a pyramid formation by undercutting a convex corner.

In an embodiment, the device comprises a plurality of matrix distributed in the transducer layer.

In an embodiment, the pH sensor matrix comprises 3 to 5 mm of length.

In an embodiment, the pH sensors matrix comprises 3 to 5 mm of width.

In an embodiment, the pH sensor height ranges from 5 - 1000 µm.

In an embodiment, the pH sensor tip diameter ranges from 1 and 50 µm.

In an embodiment, the pH sensor bottom diameter ranges from 3 - 500 µm.

In an embodiment, pH sensors are made of silicon coated with electrically conductive metals selected from silver, silver chloride, gold, platinum, iridium oxide, aluminium oxide, and coated with biocompatible material selected from silicon oxide, parylene C, polyimide, or combinations thereof, or combinations thereof.

In an embodiment, the device comprises a connection layer comprising connectors for insert the fluid in the microchannel.

In an embodiment, the device further comprises a sealing layer. Preferably the sealing layer is an upper layer for sealing the device. Preferably the sealing layer is made of polymethylmethacrylate polymer (PMMA).

In an embodiment, the sealing layer comprises a plurality of tubes and connectors for regulation of the pressure and flow of the microfluidic system of the intermediate layer.

In an embodiment, the number of cavities ranges from 2 to 32.

In an embodiment, the intermediate layer comprises a micro-inlet and a micro-outlet for connecting the microfluid system with the plurality of cavities for the cell growth.

In an embodiment, the transducer layer may further comprise at least one pO₂ sensors or CO₂ sensors or NO sensors or analyte capture sensors, or metabolic sensors, or their combinations.

In an embodiment, the transducer layer may further comprise potentiometric sensors or amperometric sensors or impedance sensors or optical sensors or surface acoustic wave field sensors or their combinations.

In an embodiment, the intermediate layer is made of glass, silicon or synthetic polymers, or their combinations.

In an embodiment, the synthetic polymer is preferable transparent.

In an embodiment, the synthetic polymer is selected from polystyrene (PS), polycarbonate (PC) or polydimethylsiloxane (PDMS).

In an embodiment, the thickness of the connection layer is between 200 - 1000 µm.

In an embodiment, the connection layer is made of a transparent synthetic polymer material, preferably polydimethylsiloxane (PDMS).

In an embodiment, the base layer is made of a material selected from glass or silicon or polymethylmethacrylate polymer (PMMA) or their combination.

In an embodiment, the thickness of the connection layer is between 200 - 1000 µm.

In an embodiment, the connection layer is made of a transparent synthetic polymer material, preferably polydimethylsiloxane.

In an embodiment, the base layer and the transducer layer are fuse or laminated for forming one single layer.

In an embodiment, the device further comprises a reservoir system.

In an embodiment, the organoid cells are three-dimensional cell aggregates.

In an embodiment, the device is a multiorgan-on-a-chip.

In an embodiment, the device to produce an organoid cell culture or cultures of the present disclosure, preferably a MoC device, may comprise:
- a base layer;
- a transducer layer containing micro(bio)sensors, heaters, photodiodes and the microelectronics for reading the sensors signals and for actuating the heating systems; being the transducer layer arranged on top of the base layer; or optionally the base layer and the transducer layer are fused forming one single layer;
- an intermediate layer arranged on the transducer layer, said intermediate layer comprises the biological models and the autonomous microfluidic system;
- a connection layer containing connectors for the fluid movement and superimposed on the intermediate layer; and
- a sealing layer;

so that, the base layer provides solid support for the additional layers;
the transducer layer providing support for housing the multiplexed micro(bio)sensor and heating systems and the microelectronics for readout, control and actuation;
the intermediate layer configured to accommodate a multiplicity of individual organs, defined by organoid cavities that are representative of an organ, comprises also a micro-inlet and a micro-outlet in communication with the microfluidic system. Each organ cavity comprises at least one organ configured to represent, but not limited to, the organs of the liver, pancreas, spleen, kidney, heart, lungs, brain and small intestine, which may represent models of healthy and/or diseased organs;
the connection layer is configured to seal and/or stabilize the organ layer, still allowing the exchange of fluids between the cavities and sections of organ growth and recirculation of the microfluidic system; and
the sealing layer is configured to comprise a multiplicity of tubes and connectors that allow regulating the pressure and flow of the microfluidic system and/or part of it, on an equivalent of selected organs. Additionally, this layer allows the incorporation of a screw-nut system, which allows the non-permanent sealing of the MoC device, assisting in accessing biological models whenever necessary.

In the present disclosure, a biological model is an experimental system that recreate aspects of human tissue function or disease, namely an organoid.

Another aspect of the present disclosure refers to the use of the multiorgan-on-a-chip device as a monitor for physicochemical parameters of long-term organoid cell culture.

Additional details and embodiments of the disclosure are defined in the specification below and in the claims.

### BRIEF DESCRIPTION OF THE DRAWINGS

The following figures provide preferred embodiments for illustrating the disclosure and should not be seen as limiting the scope of invention.
**Figure 1****:** Schematic representation of an embodiment of a multiorgan-on-a-chip (MoC) comprising a base layer **1,** a transducer layer comprising micro(bio)sensors, heating and microelectronics **2,** a layer for the microfluidic system and tissue organs growth **3,** a connection layer **4** and a sealing layer **5.**
**Figure 2****:** Schematic representation of an embodiment of an advance Organ-on-a-chip (OoC) device with microfluidic system, micro(bio)sensors and heating **6,** without organoid **6A** and with organoid **6B** into the microfluidic chamber.

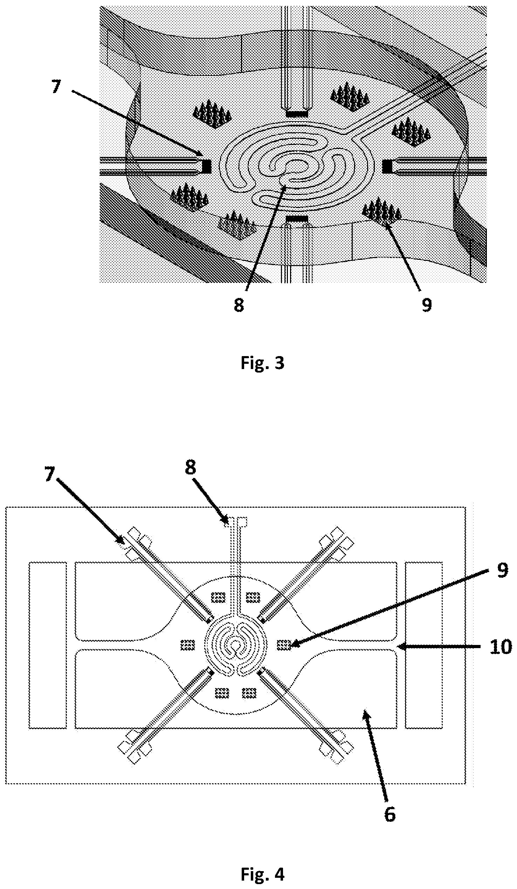

**Figure** 3: Schematic representation of an embodiment of a microfluidic chamber, comprising a temperature sensor **7,** a heating system **8,** and a pH sensor array **9.**
**Figure 4****:** Top view of a schematic representation of a multi-organ-on-a-chip device **6** comprising a temperature sensor **7,** a heating system **8,** a pH sensor array **9,** and a microchannel **10.**
**Figure 5****:** Schematic detail of an embodiment of the (bio)sensors used in the microfluidic device, showing a platinum resistance temperature detector (RTD) **11** from the temperature sensor, and the micro-needle **12** present in the pH sensor array.
**Figure 6****:** Schematic representation of an embodiment of the microfluidic system **10** with optical pH sensor **13,** build-in in a 1 mm microfluidic chamber **14.**
**Figure 7****:** Schematic representation of an embodiment of an engineering of 3D organ models, **a** diagram of the formation procedure to develop spheroids, **b** top view of micro-wells cultured with representative cell lines after 5 days of incubation, c individual and non-encapsulated spheroids in cell culture media after 5 days of incubation in micro-wells structures, **d** schematic representation of spheroids encapsulated with hydrogel, forming mimicking and representative 3D organ models.
**Figure 8****:** A representation of different embodiments of fabricated temperature microsensors with different windings (line width and spacing of 10 µm).
**Figure 9****:** Illustration of performance of the temperature microsensors with 5, 8, 12, 16, 20 and 24 windings. Grey lines represent an increase temperature from 35 to 45°C and black lines a decrease temperature from 45 to 35°C.
**Figure 10****:** Illustration of performance of the temperature sensibility with the winding number.
**Figure 11****:** Schematic representation of different embodiments for a heating system.
**Figure 12****:** Simulation results of the heating system showing the different localized heating for each embodiment (Fig. 12A and Fig. 12B).
**Figure 13****:** Illustration of the block diagram of an embodiment of an electronic circuit for control and readout the temperature microsensors signals.
**Figure 14****:** Transmittance and absorbance values at 560 nm for cell culture medium in the pH range 5.0 - 8.5, obtained with a spectrophotometer.
**Figure 15****:** Schematic representation of an embodiment of a microfluidic flow control system, **15,** connect to the MoC, **16.**
**Figure 16****:** Representation of an embodiment of heater 1 where a) the heater is without power, so no heating, and b) the heater with a current of 250 mA flowing through the heater, heating the actuator.
**Figure 17****:** Representation of an embodiment of a schematic representation of the overall geometry of the temperature microsensors.

### DETAILED DESCRIPTION

The present disclosure relates to a multiorgan-on-a-chip (MoC) device for producing a long-term organoid cell culture and the use thereof. The present disclosure comprises an advanced microfluidic system - multiorgan-on-a-chip (MoC) device - for the validation and study of nanomaterials, drugs, or mixtures thereof. The multiorgan-on-a-chip (MOC) device of the present disclosure may be used in biomedical and/or pharmaceutic applications.

The present disclosure relates to a multiorgan-on-a-chip (MoC) device for long-term organoid cell culture comprising: a base layer for supporting the device; a transducer layer arranged on the base layer; an intermediate layer comprising a plurality of cavities for organoid cell growth and a microchannel network for flowing a fluid; and a connection layer comprising connectors for insert the fluid in the microchannel; wherein the transducer layer comprises a heating device and at least a plurality of sensors in each cavity for cell control and growth; wherein each sensor is configured to measure an organoid cell growth parameter in interior of each cavity.

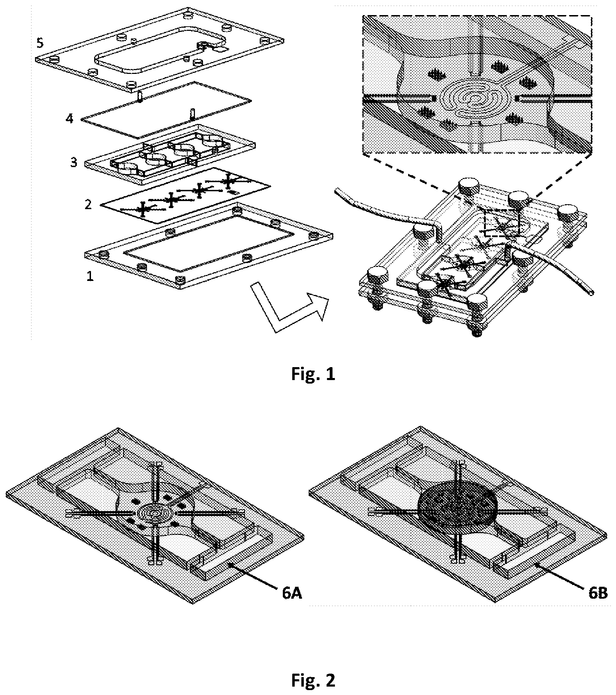

In an embodiment, **Figure 1** shows a schematic representation of a multiorgan-on-a-chip (MoC) device composed of layers with different functionalities, sealed non-permanently, which allows better handling and control of the final device.

In an embodiment, the base layer **1** is configured to function as a solid support for the additional layers of the multiorgan-on-a-chip (MoC) device of the present disclosure. Preferred materials for the base layer comprise glass, silicon or polymethylmethacrylate polymer (PMMA), or a combination of these materials.

In another embodiment, the base layer **1** can be merged with the transducer layer **2,** forming one single layer with both the functionalities of supporting the additional layers and performing the functionalities of the transducer layer. Preferably, this base layer is made of a material that enhances this double application, such as, but not limited to, silicon or glass.

In an embodiment, the incorporation of sensors and heaters can be set on the transducer layer **2,** allowing in-situ monitoring of human organ models in culture. Thus, the transducer layer can comprise one or more sensors configured and arranged in order to measure physiological parameters and homeostasis of one or more of the equivalents of organs, growth sections, complemented with integrated sensors in the microfluidic system to monitor the feeding fluid. The sensors that can be used include, but are not limited to, pH sensors or temperature sensors or pO₂ sensors or CO₂ sensors or NO sensors or analyte capture sensors, or metabolic sensors, or their combinations and may include potentiometric sensors or amperometric sensors or impedance sensors or optical sensors or surface acoustic wave field sensors or their combinations. Preferably, the transducer layer comprises at least the following set of sensors, **Figure 2****:**
- set of pH sensors arranged and distributed, preferably in direct contact with the organoid that serves as a model for the human organ. These sensors have a micro-needle or pyramidal geometry, with a tip diameter between 1 and 50 µm to avoid the perforation of the organoid models or of the hydrogels, arranged in matrix formation **12,** having each pyramidal a height between 5 and 1000 µm and a bottom diameter between 3 and 500 µm, but not limited to these dimensions. The matrix formation has a length between 3 and 5 mm and a width between 3 and 5 mm, but not limited to these dimensions. This geometry allows to monitor the internal pH of the organoids that serve as diseased and/or healthy organ models, assisting in the validation and screening of nanomaterials developed for the controlled release of drugs, by changing pH and/or temperature, or drug studies. The preferred materials for these sensors are silicon coated with electrically conductive metals, such as silver (Ag), silver chloride (AgCl), gold (Au), platinum (Pt), iridium oxide (IrO₂), aluminium oxide (Al₂O₃), among others, coated with biocompatible material, such as silicon oxide, parylene C, polyimide, among others;
- set of planar temperature microsensors **11** arranged in direct contact with the organoid that serves as a model for the human organ. These temperature microsensors **11** are based on resistance temperature detectors (RTDs) which explore the variation in electrical resistance caused by temperature changes, presenting an electrical resistance value for each absolute temperature. The small size of RTDs area, ranging from 0.076-0.38 mm², as well as their ease of integration with micro-components make them excellent candidates for applications in microfluidic devices, such as OoC. The geometry of the RTD consists of a certain number of windings (5 - 24) in a meander shaped resistor of a thin metallic film. RTDs lines width, ranging from 10 µm - 50 µm, lines length from 190 µm - 950 µm, lines spacing between 10 µm - 50 µm and a film thickness from 50 nm - 200 nm. The manufacturing process of these microsensors is based on deposition technologies for thin metallic films, such as physical vapor deposition (PVD), and their standardization is carried out by photolithography techniques, physical corrosion, chemical corrosion, among others. Microsensors are manufactured from several metals, including platinum (Pt), nickel (Ni), titanium (Ti), chromium (Cr), gold (Au), aluminium (Al), among others. The preferred material for RTD is platinum due to its advantages, namely, the highly linear response of its electrical resistance according to the temperature changes, high chemical resistance, high mechanical hardness, high temperature stability with a high melting point (1771.85 °C) and biocompatibility; and
- a heating system **8,** that makes it possible to radiate and supply heat to the organoid representative of the human organ model, to mimic the hyperthermia temperature (41 - 45 °C). This system allows to control the local temperature of the organoid model and, thus, to test the effect of hyperthermia temperature in synergy with the chemotherapy action promoted by nanomaterials that can function as drug nano-carriers. This system comprises a heat source based on a resistive metallic thin film filament with a meander, spiral, coil or another configuration. Preferably the configuration is spiral. The heating system **8** comprises heater lines width, ranging from 30 µm - 500 µm, lines length from 5000 µm - 50000 µm, lines spacing between 30 µm - 360 µm and a film thickness from 50 nm - 200 nm. The passage of an electric current through this filament will generate heat through the Joule effect, and the value of the applied electric current allows to control the temperature generated in the heating system. Several metals can be used to make the resistive filament; however, the preferred material is platinum (Pt). The manufacture of the heating system and RTD microsensors with the same material (platinum, for example), reduces the complexity of the manufacturing process, since the two systems can be manufactured simultaneously-.

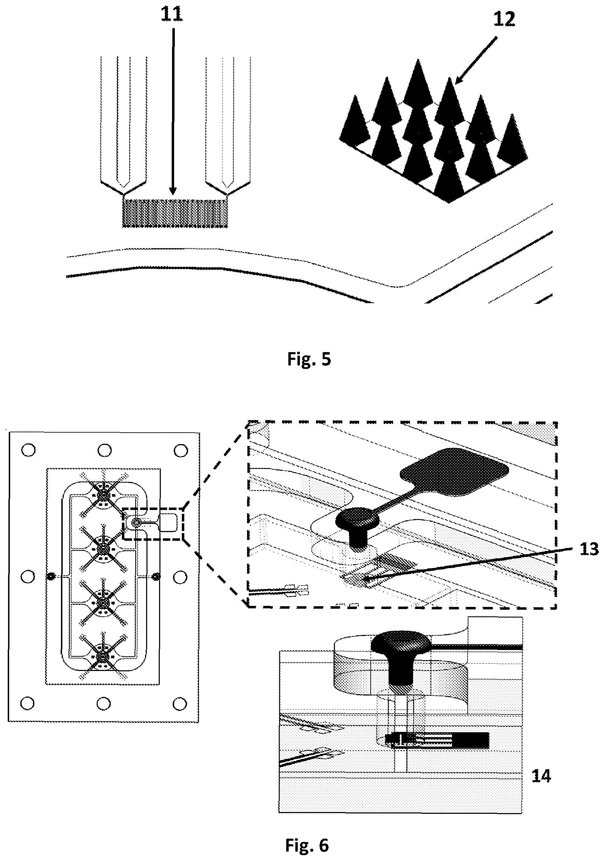

In an embodiment, where the layer **2** is of silicon, it also comprises a system of optical sensors that, through the microfluidic system, measure and monitor the conditions of homeostasis and/or the effect of drugs in studies (free form and/or encapsulated in nanomaterials), for example measuring pH, temperature, O₂, CO₂, NO, specific biomarkers released by organoids, among other analytes that complement this analysis;
- particularly, a system comprising optical pH sensors, configured and located at the microfluidic system, allowing the measure of the pH of the culture medium that mimics the blood system by analysing the colorimetric pH indicator, phenol red, added commercially to cell culture media. For this purpose, the optical pH sensor **13,** built-in at the transducer layer and the intermediate layer, **Figure 6****,** with a microchannel chamber of 1 mm length and height between 0.4 and 1.0 mm but not limited to these dimensions, comprises a light emitting diode (LED) system, which can be white or monochromatic, and a set of photodetectors, normally photodiodes, optimized for the spectral range of green and blue, which allows to measure by absorbance the pH indicator, phenol red at 560 nm, present in almost all commercial culture media formulations that serve as feed fluid, **Figure 6****.**

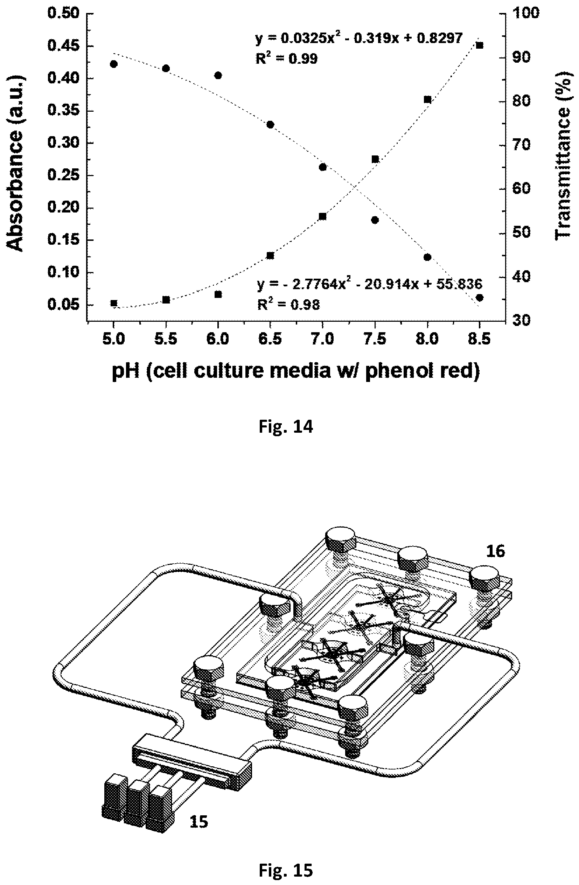

In an embodiment, as illustrate in **Figure 14****,** the optical sensor is able to measure pH differences between 5.0 and 8.5, in continuous, by using a CMOS photodiode. The photodiodes can be fabricated in CMOS and in the same layer as the transducers systems (layer **2**), if the material for this layer is silicon. Also, all the readout electronics, for incorporating the sensors signals readout, can be incorporated in the same silicon layer. On top of the photodiodes it will be deposited optical filters, by PVD, for spectral optimization.

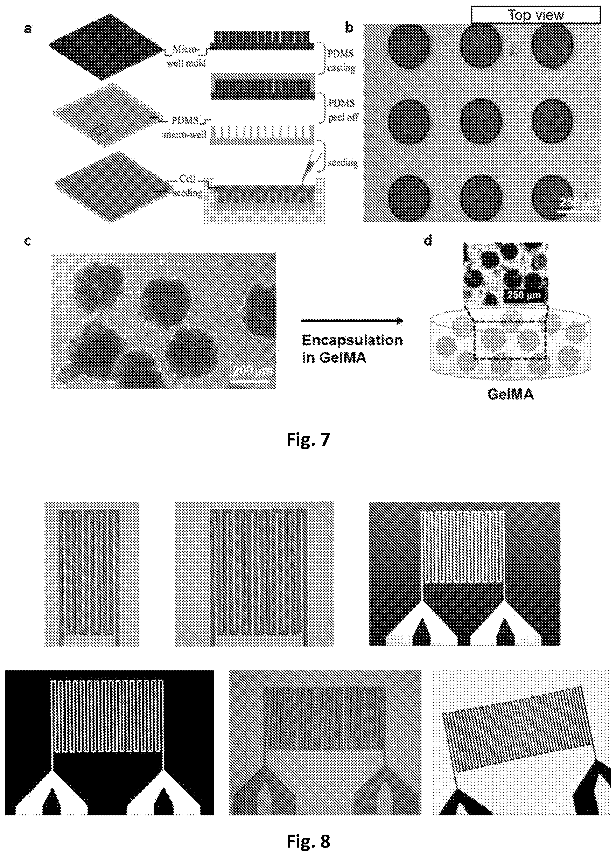

In an embodiment, the device of the present disclosure comprises an intermediate layer, **3,** located at the top of the base layer, **2.** The intermediate layer comprises one or more growth sections, configured to comprise in each organoid cavity a representation of a human organ. Each organ cavity comprises at least one organ equivalent configured to represent, but not limited to, the organs of the liver, pancreas, spleen, kidney, heart, lungs, brain and small intestine, which can represent models of healthy and/or diseased organs. In addition, the intermediate layer can comprise other organ equivalents, such as skin, circulatory system, immune system, endocrine system, female and/or male reproductive system. The intermediate layer also comprises the microfluidic system implemented for fluid circulation between organs through micro-inlet and micro-outlet systems connected by microchannel networks. The organoids described in the present disclosure are defined as three-dimensional cell aggregates, cultured *in vitro,* with dimensions up to several millimetres, and that serve as a model for the organs of a human organism, **Figure 7****.** This method is described in Lee et al., "A Heart-Breast Cancer-on-a-Chip Platform for Disease Modelling and Monitoring of Cardiotoxicity Induced by Cancer Chemotherapy", Small, 2020, 2004258 (doi: 10.1002/smll.202004258). Each organoid represents an organ model and is constituted from cell lines, which are identical, self-sufficient and representative of human organs, and can represent healthy and/or diseased tissues. The organoid models represent an exceptional opportunity for the predictive tests of the effect of nanomaterials developed as theranostic nanosystems, as well as of new drugs developed by the pharmaceutical industry for the treatment of human diseases. In the present disclosure, the MoC device comprises a multiplicity of organoid cavities with dimensions between 0.3 and 50 mm³ but not limited to these dimension, representative of an organ type, in which the size of the respective organoid model can be easily adjusted according to the respective organ type.

In an embodiment, at least one of the following models (or organoids) may be produce in the device of the present disclosure:
- the liver organoid, which can be a liver lobe with a volume of 1.6 to 16.0 mm³;
- the pancreatic organoid, which can be an islet of Langerhans, with a volume of 1.1 to 10.5 mm³;
- the spleen organoid, which can be white and red pulp tissue with a volume of 0.4 to 4.0 mm³;
- the kidney organoid, which can be tissue from nephrons with a volume of 0.3 to 3.0 mm³;
- the heart organoid, which can be cardiomyocyte tissue with a volume of 0.6 to 5.8 mm³;
- the brain organoid, which can be tissue from the cerebral cortex with a volume of 0.5 to 5.0 mm³.

In the present disclosure, the representation of each organ is mimicked through the proportionality of the size of each organ in the organism, which can be defined by a proportionality factor between 0.0001 (1/10 000) and 0.00001 (1/100 000).

In an embodiment, the MoC device of the present disclosure may comprises in each cavity: 1 liver organoid, 1 pancreas organoid, 1 spleen organoid, 2 kidney organoids, 1 heart organoid, 10 brain organoids.

In another embodiment, the device of the present disclosure may comprise multiple organoids, which may include other models of organoids from other complementary organs.

In an embodiment, the intermediate layer is preferably made of glass, silicon oxide or synthetic polymers, or their combinations. Within synthetic polymers, optically transparent ones are preferred, which include, by way of example, polystyrene (PS), polycarbonate (PC) and polydimethylsiloxane (PDMS), with PDMS being the preferred material.

In an embodiment, the microfluidic system of the present disclosure is connected to the organoids cavities, **3,** in order to mimic the vascular system of a higher organism, allowing the supplement of organ models with nutrients and O₂, the interaction between different organ models and also serving as a disposal system for their metabolic products. Thus, the aforementioned microfluidic system represents a vital system for the homeostatic maintenance of the entire device, which is monitored from micro(bio)sensors integrated in it, **2.** The microfluidic flow control system, **15,** refers to the fluid that circulates in the microchannel system of the present invention from an external reservoir **(****Figure 15****).** This external reservoir system, connected by a system of micro-inlets and micro-outlets located in the connection layer **4,** has the advantage of being able to replace or collect the supply fluid autonomously, without disturbing the MoC. The microfluidic system is controlled by external micro-pumps or miniaturized micro-pumps with pneumatic actuation or miniatured piezoelectric micro-pumps integrated in the MoC device and/or controlled by integrated micro-valves. The microfluidic system can also comprise a series of microchannels and bifurcations that allow connectivity between a set of organs, for example, the digestive system, cardiovascular system, reproductive system or other. Preferably, the fluid is blood or a blood equivalent, such as a commercial cell culture medium. These advanced microfluidic architectures allow to mimic biological functions essential to higher organisms, providing the ideal platform for preclinical tests of nanomaterials developed for biomedical applications and/or drugs developed for the treatment of human diseases.

In an embodiment, the connection layer **4,** superimposed on the intermediate layer **3,** allows to seal and/or stabilize the organ equivalents, as well as allowing the communication with the sealing layer 5. Preferably, the connection layer has a thickness of 200 - 1000 µm of a transparent synthetic polymeric material, such as, for example, PDMS.

In an embodiment, the MoC device of the present disclosure further comprises a sealing layer **5,** superimposed on the connection layer **4.** This sealing layer allows the sealing of the MoC device in a non-permanent way, through connections means, preferable a screw-nut system, and comprising a plurality of tubes and inlet/outlet connectors, arranged in order to regulate the pressure force and flow rate applied to the models of organs and the microfluidic system, and/or part of it. Preferably, the sealing layer is made of glass or synthetic polymers. Within synthetic polymers, optically transparent ones are preferred, which include, for example, polycarbonate (PC), polypropylene (PP) or polymethylmethacrylate (PMMA), or their combination.

In an embodiment, the present disclosure relates to a MoC device for operating potential in homeostatic conditions, assisted by a set of micro(bio)sensors in situ integrated with the organoid models for the screening and preclinical validation of nanomaterials and drugs developed for the treatment of human diseases, and a pH optical sensor integrated in the microfluidic system to monitor the cell culture media, which can be replaced when needed. The MoC can be applied in different configurations depending on the architecture of the organoids representative of the organs present in the device. In addition, the device described in this invention allows application in safety tests as immunological, infectious and/or oncological models.

### Example 1:

In an embodiment, the temperature microsensors are fabricated by lift-off techniques, with an area ranging from 0.076 to 0.38 mm² and lines width of 10 µm, lines length from 190 to 950 µm, lines spacing of 10 µm and a film thickness of 200 nm. The geometry of the temperature microsensor comprises 5 to 24 windings, but not limited to, in a meander shaped resistor of a thin metallic film **(****Figure 8****).** The following table shows an embodiment of the sensor sensitivity for sensors comprising 5, 8, 12, 16, 20 or 24 windings.

**Table I - Number of windings in serpentine-shaped (meander shaped) geometry**

| **Number of windings in the serpentine-shaped geometry** | **Width (mm)** | **Length (mm)** | **Area (mm²)** | **Sensor's sensitivity** |
|---|---|---|---|---|
| **5** | 0.4 | 0.19 | 0.076 | 0.0719 |
| **8** | 0.4 | 0.31 | 0.124 | 0.1126 |
| **12** | 0.4 | 0.47 | 0.188 | 0.1683 |
| **16** | 0.4 | 0.63 | 0.252 | 0.2258 |
| **20** | 0.4 | 0.79 | 0.316 | 0.3052 |
| **24** | 0.4 | 0.95 | 0.38 | 0.3542 |

In an embodiment, the fabrication process started with the cleaning of a silicon or glass wafer using the PVA Tepla GIGAbatch 360M equipment comprising a microwave plasma, created from a mixture of O₂ and CF₄, operating at 2.45 GHz. Then, it is performed a double layer lift-off process using standard photolithography and wet etching, using MicroChemicals AZ-family photoresist, developer, and solvents. Lift-off resist (LOR1A-SB200) and positive photoresist AZ4110 are sequentially deposited in the substrate by spin coating and cured at 200°C during 3 min and at 110°C during 1 min, respectively. A 405 nm high-resolution laser is used to expose the photoresist for substrate patterning by a direct writer laser process (DWL2000, Heidelberg Instruments) and a development step using AZ400K solvent during 4.5 min is performed. After this stage, a physical vapour deposition was used for performing two sputtering processes. For this purpose, a Kenosistec ultra-high vacuum magnetron sputtering system consisting in a confocal geometry, was employed to deposit an adhesion layer of tantalum with a thickness of 5 nm and a layer of platinum with a thickness of 200 nm. Once the above steps have been successfully carried out, the lift-off resist is removed using a 60°C pre-heated solution of mr-REM500 (microresist technology GmbH) in an ultrasonic bath to improve the etching rate. This etching step is performed during 150 min. Finally, photoresist residues in the wafer with the patterned sensors are removed by a high temperature plasma ashing process during 20 min. An electrical insulator with high thermal conductivity is further deposited on top of the temperature microsensors for electrically isolate the sensors from the fluidic medium, increase chemical and mechanical resistance and provide a rapid response to changes in temperature. A conformal layer of SiO₂ or Al₂O₃, is deposited by chemical vapour deposition or atomic layer deposition, respectively. Then, a high-resolution mask aligner (MA6BA6 Suss Microtec) is used to transfer a mask to the wafer. For that, photoresist AZ4110 is spin-coated and cured at 110°C during 1 min on the wafer, which is then exposed at a wavelength of 365 nm and developed using AZ400K solvent. A dry etching of the SiO₂ using a reactive ion etching system (SPTS APS) or a wet etching process using aluminium etchant 16:1:2 W/AES (Fujifilm, Electronic Materials) is performed in order to remove the deposited material merely in the regions where electrical contacts are made. At the end, a plasma ashing step is performed for removing the remaining photoresist. In a further embodiment, temperature measurements with the fabricated sensors were performed. The apparatus used for testing the temperature microsensors comprises a current source for supplying the sensors, a hotplate as a heating source for control the temperature in the sensors, a multimeter to read the electric signals from the sensors and an infra-red camera to confirm the temperature changes. The supply current is 100-500 µA, since higher current originate an error known as self-heating.

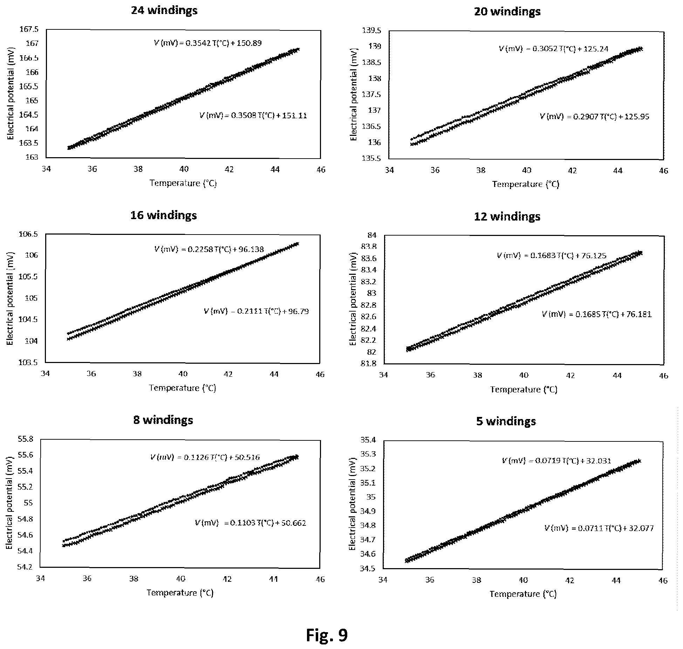

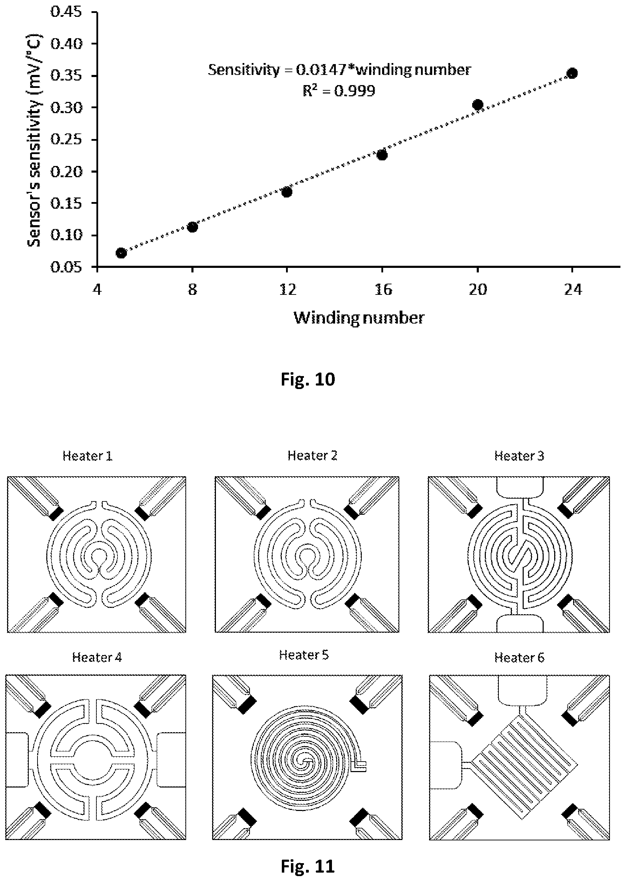

In an embodiment, the temperature microsensors performance with 5, 8, 12, 16, 20 and 24 windings were obtained, considering the sensibility and linearity **(****Figure 9****).** The results showed a linear increase in the temperature sensibility with the windings number **(****Figure 10****).**

In an embodiment **Figure 9** shows the RTDs response. It is evident a high linearity between temperature and electrical potential. Furthermore, when the number of windings is increased, the sensitivity also increases linearly (cf. **Figure 10****).** The experimental sensor's sensitivity ranges from 0.0719 to 0.3542 mV/°C for 5 to 24 windings. In an embodiment, since the higher sensitivity is obtained for the 24 windings sensor, ^{~}35 µV for a step of 0.1 °C, this geometry was the chosen one for being integrated in the organ-on-a-chip.

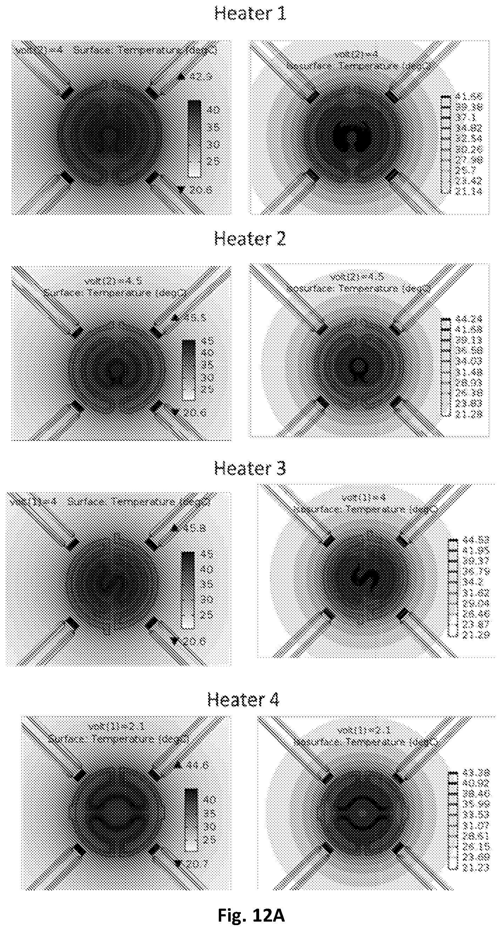

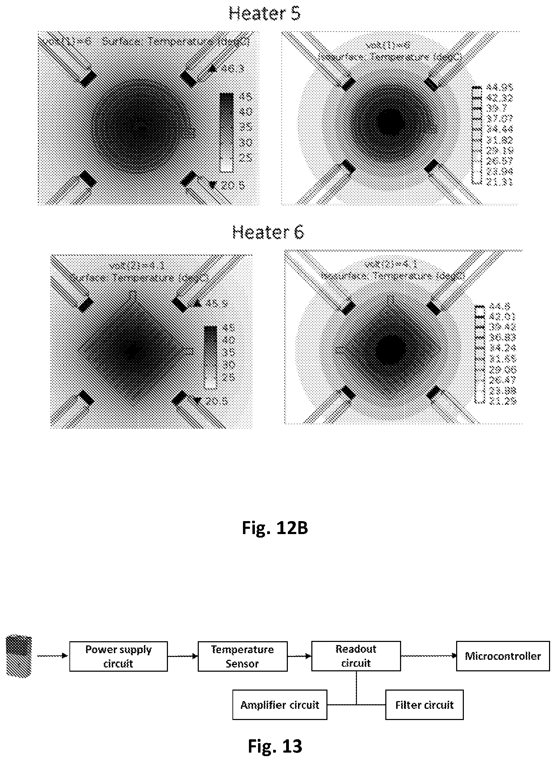

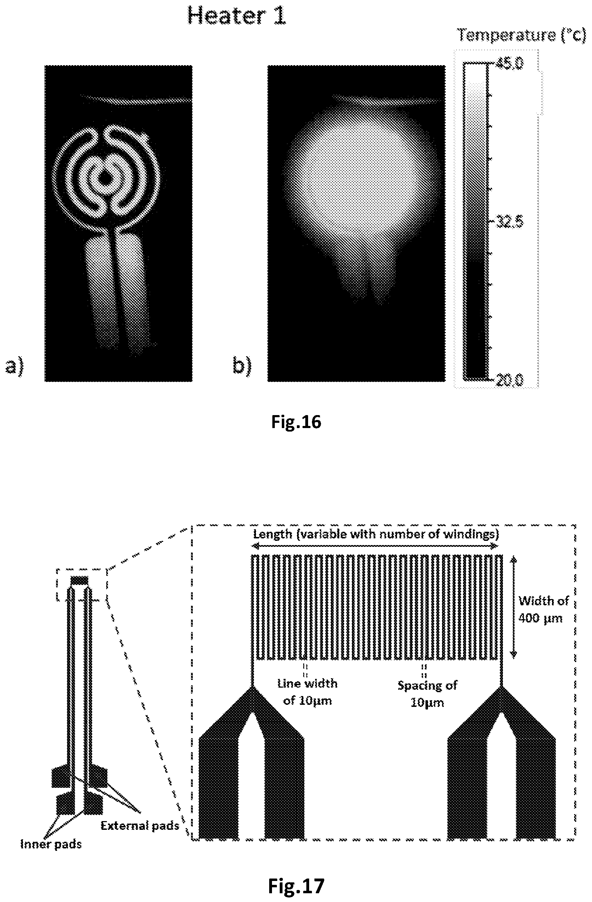

In an embodiment, the heating system is fabricated simultaneously with the temperature sensors using the same fabrication process. The geometry of the heaters comprises a resistive metallic thin-film in a meander, spiral, coil or another configuration **(****Figure 11****).** Numerical simulations of five resistive micro-heaters were performed and the temperature distribution was analysed **(****Figure 12A** **and** **Figure 12B****).** All of them have potential to promote a heating system in the temperature range of 35 to 45 °C, but the heater 1 provides a higher uniformity in the temperature distribution with a variation of <2 °C (between the periphery and the central region, **Figure 12A** **and** **Figure 12B****). Table 1** shows the simulation results of the temperature obtained in each heater using several electrical voltages. **Figure 16** shows the experimental heating profile of heater 1, acquired with an infra-red camera, a) without power, so no heating, and b) with a current of 250 mA flowing through the heater, heating the actuator.

In an embodiment, the temperature sensors include a power supply and an electronic circuit for control and readout **(****Figure 13****).** An adjustable current source (for example the LM134) was used to provide a precise current of 100-500 µA for excite the sensors. This component was chosen because allows adjust the current from 1 µA to 10 mA with high accuracy and low-noise. Any fluctuation in the excitation current will originate a change in the electrical potential of the sensors. So, the precision of the current source is crucial in the performance of the temperature measurements, especially when a high resolution is needed (0.1°C). The implementation of a high-sensitivity and noise-free reading circuit capable of making viable readings is required. So, an electronic circuit, preferably based on a 4-wire configuration or Wheatstone bridge, but not limited to, was implemented, connected to an amplifier, for example, the INA126 from Texas Instruments, for amplifying the sensors signals and converting them into a usable voltage level for the microcontroller's Analog Digital converter (ADC), and further stored in a SD or present in a display.

In an embodiment, **Fig. 17** schematically depicts the meander-shaped structure of one of temperature microsensors.

In an embodiment, the geometry of the RTD studied consist of meander-shaped windings (5 - 24) of a 200 nm platinum thin film with a line width and spacing of 10 µm. The width was fixed to 400 µm and the length is variable as function of the windings number.

In an embodiment, these microsensors were designed in a four-wire configuration, where the excitation is driven through the external pads and the electrical potential is measured in the inner pads. This configuration allows read the electrical potential with high accuracy without interference from the lead wires used for the sensor excitation.

Table II shows the simulation results of the average temperature obtained in the heater using several electrical voltages.

**Table II - Results of the average temperature obtained in the heater using several electrical voltages.**

| **HEATER** | **ELECTRIC POTENTIAL (V)** | **T AVERAGE HEATER (DEGC)** |
|---|---|---|
| **1** | 3.5 | 35.850 |
| | 4 | 40.258 |
| | 4.5 | 45.055 |
| **2** | 4 | 36.564 |
| | 4.5 | 40.551 |
| | 5 | 44.845 |
| **3** | 3.5 | 36.461 |
| | 4 | 41.036 |
| | 4.5 | 45.968 |
| **4** | 1.8 | 35.730 |
| | 2.1 | 40.862 |
| | 2.4 | 46.532 |
| **5** | 5 | 35.376 |
| | 6 | 41.478 |
| | 6.7 | 46.179 |
| **6** | 3.6 | 36.487 |
| | 4.1 | 40.838 |
| | 4.6 | 45.739 |

In an embodiment, the simulations and experimental results show that one of the best geometries for temperature sensor is a platinum meander-shaped RTD with 24 windings, featuring 200 nm platinum film thickness, 10 µm of line width and spacing and a RTD area of 0.38 mm². Moreover, the performance of the microsensor results in high sensitivity to temperature changes, ~0.3542 mV/°C. The small size of each sensor allows integrate multiple sensors in a microfluidic chamber for real-time temperature measurements with high precision. In an embodiment, the sensor also allows to evaluate the temperature of the microenvironment, which add essential information for assessing the efficiency of drug nanocarriers triggered by mild temperature within the OoC.

The pH sensor was fabricated with the following method.

In an embodiment, the pyramidal microstructures for the pH sensors are obtained from a silicon wafer, with {100}-crystal orientation for wafer surface plane, by using an anisotropic silicon wet etch process with potassium hydroxide (KOH).

In an embodiment, the fabrication process starts with the deposition of the materials used as mask for the wet etching process on the silicon wafer surface. A silicon dioxide (SiO₂) layer followed by a silicon nitrite (SiNx) layer are deposited by plasma enhanced chemical vapour deposition. The wafer is then exposed to hexamethyldisilazane (HDMS) at 150 °C during 5 min in order to remove the moisture and promote the photoresist adhesion. A positive photoresist is then spin-coated and baked on the wafer surface, to be patterned by means of optical lithography. After development for removing the exposed resist, the mask pattern is transferred to the oxide/nitrite bilayer by means of a reactive ion etching process. The resist mask is finally removed using a plasma ashing process, and the subsequent wet etching process is performed using a KOH aqueous solution for the definition of the silicon microstructures.

In an embodiment, the pyramid formation is based on convex corner undercutting, where local fast etching crystal planes are revealed. The process starts with the formation of {111}-silicon crystal planes. After a certain etch depth, {111}-crystal planes are etched away by faster etching planes with an octagon at the base. The pyramid shape is formed when 8 high index crystal planes, revealed as {312} planes, come together on top of the frustum generating a sharp tip. At this stage, the remaining mask will become detached. When the desired pyramid height is reached the wafer is rinsed in deionized water in order to stop the etching process, and then dried out with a nitrogen blow.

In an embodiment, the aspect ratio of the pyramid height to the bottom diameter of the high index crystal planes is 3:2. With a detailed understanding of the convex corner undercut towards the pyramid formation, mask sizes can be computed for any desired height. Preferably, both KOH concentration (between 5% (wt/wt) and 50% (wt/wt)) and temperature have to be selected for a more precise control of the etch rates in the different crystal orientations. This fabrication process provides pyramidal microstructures on a flat surface defined by {100}-crystal orientation. In subsequent steps, a physical vapour deposition process can be used to deposit electrically conductive metals, such as, but not limited to, silver, silver chloride, gold, platinum, iridium oxide, aluminium oxide. And a final deposition using chemical vapour deposition or a spin-coating process can be used for depositing a layer of a biocompatible material, such as silicon oxide, parylene C or polyimide.

The integration of the sensors in the microfluidic device will be now disclosed.

In an embodiment, the fabrication process used for the pyramidal microstructures provides a quite flat bottom surface, defined by planes with {100}-crystal orientation. If KOH concentration and temperature are properly selected the surface roughness achieved will allow the monolithic integration of the metallic heaters and temperature sensors. After fabrication of the pH sensors and metallic structures on the silicon substrate, it will be possible to integrate all these devices into a microfluidic platform by creating the microfluidic channels with a SU-8 lithography process, or by means of bonding to a PDMS microfluidic layer with previously casted channels.

In an embodiment, small surface roughness (~10 nm) can be achieved for surfaces with {100} silicon crystal orientation if relatively high HOH concentrations (>30%) and temperatures (>70 °C) are used, allowing a subsequent monolithic integration of the metallic heaters and temperature sensors.

The present disclosure comprises an advanced microfluidic system for the validation and study of nanomaterials, drugs, or mixtures thereof, intended to be used in biomedical and/or pharmaceutic applications. The present disclosure relates to a device for the production of an organoid cell culture or cultures comprising: a base layer for support; a transducer layer arranged on the base layer; an intermediate layer, arranged on the transducer layer, comprising a plurality of openings to form openings to form cavities for organoid cell growth and a plurality of slits to form microchannel network for fluid flow; wherein the transducer layer comprises a heating device and at least a plurality of sensors in each cavity for cell parameter control and growth; wherein the each sensor is located to measure a organoid cell growth parameter in the interior of each cavity.

## Claims

1. A device for the production of an organoid cell culture or cultures, comprising:
a base layer (1) for support;
a transducer layer (2) arranged on the base layer (1); and
an intermediate layer (3), arranged on the transducer layer (2), comprising a plurality of openings to form cavities for organoid cell growth and a microchannel network for fluid flow;
wherein the transducer layer (2) comprises a heating device (8) and at least a plurality of sensors in each cavity for cell parameter control and growth;
wherein each sensor is located for measuring an organoid cell growth parameter in the interior of each cavity;
wherein the heating device (8) is located in the interior of each cavity;
wherein the heating device (8) and the plurality of sensors are in direct contact with the organoid cell culture or cultures;
wherein the plurality of sensors is selected from temperature sensor (7), pH sensor (9) or combinations thereof.

2. The device according to the previous claim wherein the temperature sensors (7) are resistance temperature detectors.

3. The device according to any of the previous claims wherein the number of temperature sensors (7) in each cavity ranges from 1 to 48, preferably 4 to 16.

4. The device according to any of the previous claims wherein the temperature sensor (7) has meander shaped resistor of a metallic film, preferably the meander shape comprises 5 to 72 windings, preferably 5 to 24.

5. The device according to any of the previous claims wherein the configuration of the heating device (8) is selected from meander, spiral, coil; preferably a spiral and/or the heating device is made of a metallic film filament, preferably wherein the metallic film is platinum.

6. The device according to any of the previous claims wherein the plurality of pH sensors (9) is grouped and wherein each cavity comprises several groups of a plurality of pH sensors (9), preferably the plurality of pH sensors (9) is grouped for forming a matrix, more preferably the groups of a plurality of pH sensors (9) are distributed uniformly in the cavity .

7. The device according to any of the previous claims wherein the shape of the pH sensors (9) comprises a micro-needle shape (12) or, a pyramidal shape, preferably a pyramidal shape having a flat bottom surface and a sharp tip.

8. The device according to any of the previous claim 7 wherein the sharp tip of the pyramidal shape comprises 8 high index crystal planes and/or wherein the flat bottom surface comprises {100} silicon crystal orientation and/or wherein the aspect ratio of the pyramid height to the bottom diameter of the high index crystal planes is 3:2.

9. The device according to any of the previous claims wherein the pH sensors (9) comprises silicon coated with an electrically conductive metal, wherein the metal selected from a list consisting of: silver, silver chloride, gold, platinum, iridium oxide, aluminium oxide, and coated with biocompatible material selected from silicon oxide, parylene C, polyimide, and combinations thereof.

10. The device according to any of the previous claims comprises a connection layer (4) comprising connectors for insert the fluid in the microchannel and/or a sealing layer (5).

11. The device according to the previous claim wherein the sealing layer (5) comprises a plurality of tubes and connectors for regulation of the pressure and flow of the microfluidic system of the intermediate layer.

12. The device according to any of the previous claims wherein the intermediate layer (3) comprises a micro-inlet and a micro-outlet for connecting a microfluid system of the microchannel network (10) with the plurality of organoid cavities for the cell growth.

13. The device according to any of the previous claims wherein the transducer layer (2) further comprises at least one pO₂ sensors or CO₂ sensors or NO sensors or analyte capture sensors, or metabolic sensors, or their combinations and/or wherein the transducer layer (2) further comprises potentiometric sensors or amperometric sensors or impedance sensors or optical sensors or surface acoustic wave field sensors or their combinations .

14. The device according to any of the previous claims wherein the device is a multiorgan-on-a-chip.

15. Use of the device according to claims 1 to 14 as a monitor for physicochemical parameters of long-term organoid cell culture.

## Patentansprüche

1. Eine Vorrichtung zur Herstellung einer organoiden Zellkultur oder Kulturen, umfassend:
eine Grundschicht (1) als Träger;
eine Wandlerschicht (2), die auf der Grundschicht (1) angeordnet ist; und
eine Zwischenschicht (3), die auf der Wandlerschicht (2) angeordnet ist, umfassend eine Vielzahl von Öffnungen zur Bildung von Hohlräumen für das Wachstum organoider Zellen und ein Mikrokanalnetz für einen Flüssigkeitsstrom;
wobei die Wandlerschicht (2) eine Heizvorrichtung (8) und mindestens eine Vielzahl von Sensoren in jedem Hohlraum zur Kontrolle der Zellparameter und des Wachstums umfasst;
wobei jeder Sensor zur Messung eines organoiden Zellwachstumsparameters im Inneren eines jeden Hohlraums angeordnet ist;
wobei sich die Heizvorrichtung (8) im Inneren eines jeden Hohlraums befindet;
wobei die Heizvorrichtung (8) und die Vielzahl von Sensoren in direktem Kontakt mit der oder den organoiden Zellkulturen stehen;
wobei die Mehrzahl der Sensoren aus Temperatursensoren (7), pH-Sensoren (9) oder Kombinationen davon ausgewählt ist.

2. Die Vorrichtung nach dem vorangehenden Anspruch, wobei die Temperatursensoren (7) Widerstandstemperatursensoren sind.

3. Die Vorrichtung nach einem der vorangehenden Ansprüche, wobei die Anzahl der Temperatursensoren (7) in jedem Hohlraum zwischen 1 und 48, bevorzugt zwischen 4 und 16 liegt.

4. Die Vorrichtung nach einem der vorangehenden Ansprüche, wobei der Temperatursensor (7) einen mäanderförmigen Widerstand aus einem Metallfilm aufweist, wobei die Mäanderform bevorzugt 5 bis 72 Windungen, bevorzugt 5 bis 24, aufweist.

5. Die Vorrichtung nach einem der vorangehenden Ansprüche, wobei die Konfiguration der Heizvorrichtung (8) ausgewählt ist aus Mäander, Spirale, Spule; bevorzugt eine Spirale und/oder die Heizvorrichtung aus einem Metallfilmfilament hergestellt ist, wobei der Metallfilm bevorzugt Platin ist.

6. Die Vorrichtung nach einem der vorangehenden Ansprüche, wobei die Vielzahl der pH-Sensoren (9) gruppiert ist und wobei jeder Hohlraum mehrere Gruppen einer Vielzahl von pH-Sensoren (9) umfasst, wobei die Vielzahl von pH-Sensoren (9) bevorzugt gruppiert ist, um eine Matrix zu bilden, wobei die Gruppen einer Vielzahl von pH-Sensoren (9) besonders bevorzugt gleichmäßig in dem Hohlraum verteilt sind.

7. Die Vorrichtung nach einem der vorangehenden Ansprüche, wobei die Form der pH-Sensoren (9) die Form einer Mikronadel (12) oder einer Pyramide umfasst, bevorzugt die Form einer Pyramide mit einer flachen Bodenfläche und einer scharfen Spitze.

8. Die Vorrichtung nach einem des vorangehenden Anspruchs 7, wobei die scharfe Spitze der Pyramidenform 8 hochbrechende Kristallebenen umfasst und/oder wobei die flache Bodenfläche {100} Siliziumkristallorientierung umfasst und/oder wobei das Seitenverhältnis der Pyramidenhöhe zum Bodendurchmesser der hochbrechenden Kristallebenen 3:2 beträgt.

9. Die Vorrichtung nach einem der vorangehenden Ansprüche, wobei die pH-Sensoren (9) Silizium umfassen, das mit einem elektrisch leitfähigen Metall beschichtet ist, wobei das Metall aus einer Liste ausgewählt ist, bestehend aus: Silber, Silberchlorid, Gold, Platin, Iridiumoxid, Aluminiumoxid, und mit einem biologisch verträglichen Material beschichtet ist, ausgewählt aus Siliziumoxid, Parylene C, Polyimid und Kombinationen davon.

10. Die Vorrichtung nach einem der vorangehenden Ansprüche umfassend eine Verbindungsschicht (4), umfassend Anschlüsse zum Einführen der Flüssigkeit in den Mikrokanal und/oder eine Abdichtschicht (5).

11. Die Vorrichtung nach dem vorangehenden Anspruch, wobei die Abdichtschicht (5) eine Vielzahl von Röhren und Anschlüssen zur Regulierung des Drucks und des Flusses des mikrofluidischen Systems der Zwischenschicht umfasst.

12. Die Vorrichtung nach einem der vorangehenden Ansprüche, wobei die Zwischenschicht (3) einen Mikroeinlass und einen Mikroauslass zum Verbinden eines Mikrofluidsystems des Mikrokanalnetzes (10) mit der Vielzahl organoider Hohlräume für das Zellwachstum umfasst.

13. Die Vorrichtung nach einem der vorangehenden Ansprüche, wobei die Wandlerschicht (2) ferner mindestens einen pO₂-Sensor oder CO₂-Sensor oder NO-Sensor oder einen Analyten-erfassenden-Sensor oder einen Stoffwechselsensor oder deren Kombinationen umfasst und/oder wobei die Wandlerschicht (2) ferner potentiometrische Sensoren oder amperometrische Sensoren oder Impedanzsensoren oder optische Sensoren oder Feldsensoren für akustische Oberflächenwellen oder deren Kombinationen umfasst.

14. Die Vorrichtung nach einem der vorangehenden Ansprüche, wobei die Vorrichtung ein Multiorgan-On-A-Chip ist.

15. Verwendung der Vorrichtung nach einem der Ansprüche 1 bis 14 als Monitor für physikochemische Parameter einer organoiden Langzeitzellkultur.

## Revendications

1. Un dispositif pour la production d'une culture ou de cultures de cellules organoïdes comprenant :
une couche de base (1) pour le support ;
une couche de transducteur (2) disposée sur la couche de base (1) ; et
une couche intermédiaire (3), disposée sur la couche de transducteur (2), comprenant une pluralité d'ouvertures pour former des cavités pour la croissance de cellules organoïdes et
un réseau à microcanaux pour l'écoulement de fluides ;
dans lequel la couche de transducteur (2) comprend un dispositif de chauffage (8) et au moins une pluralité de capteurs dans chaque cavité pour le contrôle des paramètres et la croissance des cellules ;
dans lequel chaque capteur est placé pour mesurer un paramètre de croissance de cellules organoïdes à l'intérieur de chaque cavité ;
dans lequel le dispositif de chauffage (8) est situé à l'intérieur de chaque cavité ;
dans lequel le dispositif de chauffage (8) et la pluralité de capteurs sont en contact direct avec la culture ou les cultures de cellules organoïdes ;
dans lequel la pluralité de capteurs est choisie parmi un capteur de température (7), un capteur de pH (9) ou des combinaisons de ceux-ci.

2. Le dispositif selon la revendication précédente dans lequel les capteurs de température (7) sont des détecteurs de température de résistance.

3. Le dispositif selon l'une quelconque des revendications précédentes dans lequel le nombre de capteurs de température (7) dans chaque cavité varie entre 1 et 48, préférablement entre 4 et 16.

4. Le dispositif selon l'une quelconque des revendications précédentes dans lequel le capteur de température (7) a une résistance d'un film métallique en forme de méandres, la forme de méandres comprenant préférablement 5 à 72 enroulements, préférablement 5 à 24.

5. Le dispositif selon l'une quelconque des revendications précédentes dans lequel la configuration du dispositif de chauffage (8) est choisie parmi méandres, spirale, ressort ; préférablement une spirale et/ou le dispositif de chauffage est fait d'un filament de film métallique, préférablement dans lequel le film métallique est de la platine.

6. Le dispositif selon l'une quelconque des revendications précédentes dans lequel la pluralité de capteurs de pH (9) est groupée et dans lequel chaque cavité comprend plusieurs groupes d'une pluralité de capteurs de pH (9), la pluralité de capteurs de pH (9) étant préférablement groupée pour former une matrice, les groupes d'une pluralité de capteurs de pH (9) étant plus préférablement distribués de manière uniforme dans la cavité.

7. Le dispositif selon l'une quelconque des revendications précédentes dans lequel la forme des capteurs de pH (9) comprend une forme de micro-aiguille (12) ou une forme pyramidale, préférablement une forme pyramidale ayant une surface de base plate et un sommet pointu.

8. Le dispositif selon la revendication précédente 7 dans lequel le sommet pointu de la forme pyramidale comprend 8 plans cristallins à indice élevé et/ou dans lequel la surface de base plate comprend une orientation de cristaux de silicium {100} et/ou dans lequel le rapport hauteur/largeur de la hauteur de la pyramide par rapport au diamètre de la base des plans cristallins à indice élevé est de 3:2.

9. Le dispositif selon l'une quelconque des revendications précédentes dans lequel les capteurs de pH (9) comprennent du silicium revêtu d'un métal électriquement conducteur, dans lequel le métal est choisi parmi une liste consistant en : argent, chlorure d'argent, or, platine, oxyde d'iridium, oxyde d'aluminium, et est revêtu d'un matériau biocompatible choisi parmi l'oxyde de silicium, le parylène C, le polyimide, et des combinaisons de ceux-ci.

10. Le dispositif selon l'une quelconque des revendications précédentes comprend une couche de connexion (4) comprenant des connecteurs pour introduire le fluide dans le microcanal et/ou une couche d'étanchéité (5).

11. Le dispositif selon la revendication précédente dans lequel la couche d'étanchéité (5) comprend une pluralité de tubes et de connecteurs pour la régulation de la pression et de l'écoulement du système microfluidique de la couche intermédiaire.

12. Le dispositif selon l'une quelconque des revendications précédentes dans lequel la couche intermédiaire (3) comprend une micro-entrée et une micro-sortie pour connecter un système microfluide du réseau microcanal (10) à la pluralité de cavités organoïdes pour la croissance cellulaire.

13. Le dispositif selon l'une quelconque des revendications précédentes dans lequel la couche de transducteur (2) comprend également au moins un capteur de pO₂ ou capteur de CO₂ ou capteur de NO ou capteur de capture d'analyte, ou capteur métabolique, ou une combinaison de ceux-ci et/ou dans lequel la couche de transducteur (2) comprend également des capteurs potentiométriques ou des capteurs ampèremétriques ou des capteurs d'impédance ou des capteurs optiques ou des capteurs de champs d'ondes acoustiques de surface ou des combinaisons de ceux-ci.

14. Le dispositif selon l'une quelconque des revendications précédentes dans lequel le dispositif est un multi-organe sur puce.

15. Utilisation du dispositif selon les revendications 1 à 14 en tant que moniteur de paramètres physicochimiques de culture de cellules organoïdes de long terme.
